# EUROPÄISCHE PATENTANMELDUNG

(11) **EP 0 711 552 A1**
(43) Veröffentlichungstag der Anmeldung: **15.05.1996**
(21) Anmeldenummer: 95115834.4
(22) Anmeldetag: 07.10.1995
(51) Int. Cl.: A61K 31/22, A61K 31/23

(54) **Dermatologische Zubereitungen mit einem Gehalt an Wollwachssäuren und Fettsäureglyceriden gegen Superinfektionen**

(30) Priorität: 13.10.1994 DE 4436537
(71) Anmelder: Beiersdorf Aktiengesellschaft, D-20245 Hamburg (DE)
(72) Erfinder: Wolf, Florian, Dr., D-20251 Hamburg (DE); Klier, Manfred, Dr., D-21521 Aumühle (DE); Schneider, Günther, Dr., D-20253 Hamburg (DE); Traupe, Bernd, D-22457 Hamburg (DE); Voss, Ilona, D-22457 Hamburg (DE)

(57) **Zusammenfassung**

Verwendung von Wirkstoffkombinationen aus
I) einem natürlichen Wollwachssäuregemisch oder einem durch Destillation eines natürlichen Wollwachssäuregemisches erhältlichen Wollwachssäuregemisch und
II) einer oder mehrerer Substanzen, gewählt aus der Gruppe der Monoglycerin-monocarbonsäure-monoester, der Diglycerin-monocarbonsäure-monoester und der Triglycerin-monocarbonsäure-monoester
als Wirkprinzip gegen Superinfektionen.

## Beschreibung

Die gesunde menschliche Haut ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten wurde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Darüberhinaus war es Aufgabe der vorliegenden Erfindung, Substanzen und kosmetische bzw. dermatologische Zubereitungen, solche Substanzen enthaltend, zur Verfügung zu stellen, die prophylaktisch gegen Superinfektionen wirken.

Es wurde überraschend gefunden, und darin liegt die Lösung dieser Aufgabe, daß die Verwendung von Wirkstoffkombinationen aus
(I) Einem natürlichen oder durch Destillation aufbereiteten Gemisch aus Wollwachssäuren
(II) einer oder mehrerer Substanzen, gewählt aus der Gruppe der Monoglycerin-monocarbonsäure-monoester, der Diglycerin-monocarbonsäure-monoester und der Triglycerin-monocarbonsäure-monoester
als Wirkprinzip gegen Superinfektionen, insbesondere gegen Superinfektionen der Haut, den Nachteilen des Standes der Technik abhilft.

Die erfindungsgemäßen Wirkstoffkombinationen sind hervorragend wirksam gegen Superinfektionen, insbesondere gegen solche, hervorgerufen durch Staphylokokken, Pityrosporum ovale sowie Dermatophyten.

Wollwachs oder Wollfett wird der bei der Rohwollwäsche anfallende fett- bis wachsartige Bestandteil der Rohschafwolle bezeichnet. Das Wollwachs besteht aus aus einem Gemisch von Fettsäureestern höherer Alkohole und aus freien Fettsäuren.

Die Hauptbestandteile der Wollwachssäuren sind
(a) gesättigte unsubstituierte Carbonsäuren, gemäß der Formel

   CH₃-(CH₂)ₙ-CH₂-COOH,
(b) α-Hydroxycarbonsäuren, gemäß der Formel 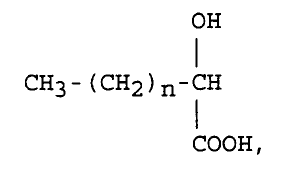
(c) ω-Hydroxycarbonsäuren, gemäß der Formel

   HO-CH₂-(CH₂)ₙ-CH₂-COOH,
(d) Isocarbonsäuren, gemäß der Formel 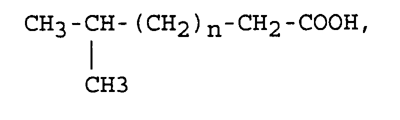
(e) α-Hydroxy-isocarbonsäuren, gemäß der Formel 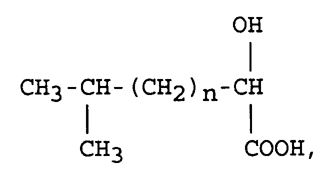
(f) ω-Hydroxy-isocarbonsäuren, gemäß der Formel 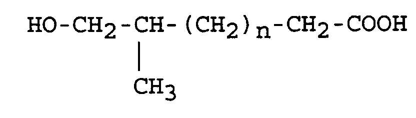
(g) Anteisocarbonsäuren, gemäß der Formel 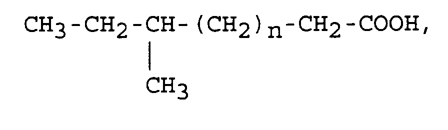
(h) α-Hydroxy-anteisocarbonsäuren, gemäß der Formel 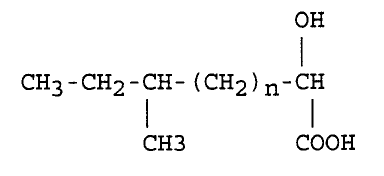
(i) ω-Hydroxy-anteisocarbonsäuren, gemäß der Formel 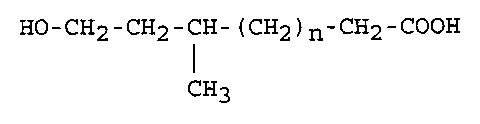
Dabei nimmt n gewöhnlich Werte von 7 - 31 an. Repräsentative Zusammensetzungen der Wollwachssäuren werden z.B. in "Parfümerie und Kosmetik", 59. Jahrgang, Nr.12/78, S.429, 430 sowie im "Lexikon der Hilfsstoffe für Pharmazie, Kosmetik und angrenzende Gebiete" von H.P.Fiedler, 1989, 3. Auflage, Editio Cantor Aulendorf, beschrieben.

Rohwollwachssäuren sind für kosmetische Zwecke nicht geeignet, statt ihrer werden für gewöhnlich destillierte Wollwachssäuren eingesetzt. Dieser Umstand und entsprechende Verfahren zur Raffinierung der Rohwollwachssäuren sind dem Fachmann bekannt.

Besonders vorteilhafte Verfahren zur Aufbereitung von Rohwollwachssäure sind beispielsweise der EP-OS 556 660 zu entnehmen.

Typischerweise bestehen Wollwachssäuren aus ca. 60 % gesättigten, unsubstituierten Carbonsäuren, ca. 30 % α-Hydroxycarbonsäuren und ca. 5 % ω-Hydroxycarbonsäuren, wobei der Rest von ca. 5 % im wesentlichen von den anderen vorgenannten Carbonsäuretypen gebildet wird.

Erfindungsgemäß besonders vorteilhaft ist eine Wollwachssäurefraktion, erhältlich durch Rohwollwachssäure durch Kurzwegdestillation im bei 10⁻¹ bar aus dem Destillationstemperaturintervall von 150 - 200° C. Der Anteil an ∝-Hydroxycarbonsäuren beträgt dabei ca. 22 - 27 %. Solche Fraktionen zeichnen sich durch folgende kennzeichnende Parameter aus:

| | |
|---|---|
| Tropfpunkt | 50 - 54° C |
| Säurezahl | 166 - 170 |
| Verseifungszahl | 175 - 190 |
| OH - Zahl | 60 - 80 |
| Jodzahl | 7 - 12 |

Zwar ist aus dem Aufsatz "Antimicrobial Factors in Wool Wax" (Australian Journal of Chemistry, 1971, 24, Seiten 153 ff.) bekannt, daß in manchen Wollwachschargen antimikrobielle Faktoren enthalten sind. Ein Hinweis in Richtung der vorliegenden Erfindung findet sich am angegebenen Orte jedoch nicht.

Die erfindungsgemäßen Monoglycerin-monocarbonsäure-monoester (in dieser Schrift gelegentlich auch Monocarbonsäuremonoglyceride genannt) werden durch die allgemeine Formel 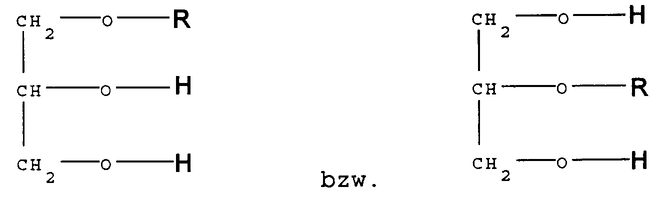
wiedergegeben, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern zugrundeliegenden Fettsäuren bzw. Monocarbonsäuren sind die
Hexansäure (Capronsäure) (R = -C(O)-C₅H₁₁),
Heptansäure (Önanthsäure) (R = -C(O)-C₆H₁₃),
Octansäure (Caprylsäure) (R = -C(O)-C₇H₁₅),
Nonansäure (Pelargonsäure) (R = -C(O)-C₈H₁₇),
Decansäure (Caprinsäure) (R = -C(O)-C₉H₁₉),
Undecansäure (R = -C(O)-C₁₀H₂₁),
Dodecansäure (Laurinsäure) (R = -C(O)-C₁₁H₂₃),
Tridecansäure (R = -C(O)-C₁₂H₂₅),
Tetradecansäure (Myristinsäure) (R = -C(O)-C₁₃H₂₇).

Besonders vorteilhaft stellt R den Octanoylrest (Caprylsäurerest) bzw. den Decanoylrest (Caprinsäurerest) dar, wird also also durch die Formeln

R = -C(O)-C₇H₁₅) bzw. R = -C(O)-C₉H₁₉

repräsentiert.

In dieser Schrift, insbesondere in den Beispielen, wird das Kürzel GMCy für Glycerinmonocaprylat und das Kürzel GMC für Glycerinmonocaprinat verwendet.

Bei den in 1-Position des Glycerins veresterten Glycerindungsgemäß aktiv und gleichermaßen von Vorteil sind die 2S- und die 2R-Konfiguration.

Es hat sich als günstig herausgestellt, racemische Gemische der Stereoisomeren zu verwenden.

In den dermatologischen Zubereitungen beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Erfindungsgemäß liegen die Di- bzw. Triglycerineinheiten der erfindungsgemäßen Diglycerin-monocarbonsäure-monoester bzw. Triglycerin-monocarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäuremonoester sind bevorzugt durch folgende Struktur gekennzeichnet (Substitutionspositionen angegeben): 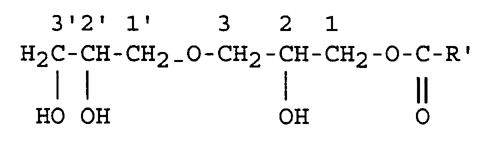
wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind bevorzugt durch folgende Struktur gekennzeichnet (Substitutionspositionen angegeben): 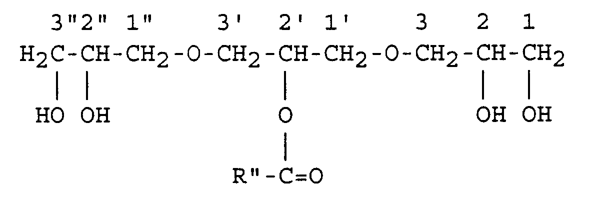
wobei R'' einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Säuren sind die
Hexansäure (Capronsäure) (R' bzw. R''= -C₅H₁₁),
Heptansäure (Önanthsäure) (R' bzw. R''= -C₆H₁₃),
Octansäure (Caprylsäure) (R' bzw. R''= -C₇H₁₅),
Nonansäure (Pelargonsäure) (R' bzw. R''= -C₈H₁₇),
Decansäure (Caprinsäure) (R' bzw. R''= -C₉H₁₉),
Undecansäure (R' bzw. R''= -C₁₀H₂₁),
10-Undecensäure (Undecylensäure) (R' bzw. R''= -C₁₀H₁₉),
Dodecansäure (Laurinsäure) (R' bzw. R''= -C₁₁H₂₃),
Tridecansäure (R' bzw. R''= -C₁₂H₂₅),
Tetradecansäure (Myristinsäure) (R' bzw. R''= -C₁₃H₂₇),
Pentadecansäure (R' bzw. R''= -C₁₄H29),
Hexadecansäure (Palmitinsäure) (R' bzw. R''= -C₁₅H₃₁),
Heptadecansäure (Margarinsäure) (R' bzw. R''= -C₁₆H₃₃),
Octadecansäure (Stearinsäure) (R' bzw. R''= -C₁₇H₃₅).

Besonders günstig werden R' und R'' gewählt aus der Gruppe der unverzweigten Alkylreste mit ungeraden C-Zahlen, insbesondere mit 9, 11 und 13 C-Atomen.
insbesondere mit 9, 11 und 13 C-Atomen.

Im allgemeinen sind die Monocarbonsäure-monoester des Diglycerins denen des Triglycerins bevorzugt.

Ganz besonders günstig sind
Diglycerinmonocaprinat (DMC) R' = 9
Triglycerinmonolaurat (TML) R'' = 11
Diglycerinmonolaurat (DML) R' = 11
Triglycerinmonomyristat (TMM) R'' = 13.

Als bevorzugter erfindungsgemäßer Monocarbonsäuremonoester des Diglycerins hat sich das Diglycerinmonocaprinat (DMC) erwiesen.

Die erfindungsgemäßen Monocarbonsäuremonoester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2''-Position Asymmetriezentren. Erfindungsgemäß aktiv und gleichermaßen von Vorteil sind die 2S2'S2''S-, die 2R2'S2''S-, die 2S2'R2''S-, die 2R2'R2''S-, die 2S2'S2''R, die 2R2'S2''R-, die 2S2'R2''R- und die 2R2'R2''R-Konfiguration.

Es hat sich als günstig herausgestellt, racemische Gemische der Stereoisomeren zu verwenden.

Die erfindungsgemäßen Zubereitungen sind besonders vorteilhaft dadurch gekennzeichnet, daß der oder die Monocarbonsäureester des Mono-, Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Es ist von Vorteil, den Gehalt an (i) Wollwachssäuregemisch und (ii) Mono-, Di- und/oder Triglycerin-monocarbonsäure-monoester so zu wählen, daß Verhältnisse (i) : (ii) wie 5 : 1 bis 1 : 20, insbesondere wie etwa 1 : 10, ganz besonders vorteilhaft wie etwa 1 : 5 entstehen.

Es ist insbesondere von Vorteil, den Gehalt an (i) Wollwachssäuren und (ii) Diglyerinmonocaprinat und/oder Glycerinmonocaprylat und/oder Glycerinmonocaprinat so zu wählen, daß Verhältnisse von (i) : (ii) wie 5 : 1 bis 1 : 20 entstehen. Insbesondere günstig im Sinne der vorliegenden Erfindungen sind Verhältnisse von (i) : (ii) wie 5 : 1 bis 1 : 5, insbesondere wie etwa 1 : 1, ganz besonders vorteilhaft wie etwa 1 : 3.

Erfindungsgemäße Zubereitungen, die erfindungsgemäßen Wirkstoffkombinationen enthaltend, sind besonders vorteilhaft dadurch gekennzeichnet, daß die erfindungsgemäßen Wirksstoffkombinationen in Konzentrationen von 0,05 - 10,00 Gew.-%, bevorzugt 0,1 - 5,0 Gew.-%, vorliegen, jeweils bezogen auf das Gesamtgewicht der Zubereitungen.

Erfindungsgemäße dermatologische Zubereitungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Crèmes oder Lotionen. Weiterhin können die Zubereitungen vorteilhaft in Form von Tinkturen, Shampoos, Wasch-, Dusch- oder Badezubereitungen oder Pudern vorliegen.

Als übliche kosmetische Trägerstoffe zur Herstellung der erfindungsgemäßen dermatologischen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen dermatologischen Zubereitungen, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen dermatologischen Zubereitungen, deren pH-Wert vorzugsweise z.B. durch übliche Puffergemische auf 4,0 bis 7,5 insbesondere 5,0 bis 6,5, eingestellt wird, Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B.α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ -Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist für die vorliegende Erfindung vorteilhaft, daß der pH-Wert der erfindungsgemäßen dermatologischen Zubereitungen kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Vorteilhaft wird der pH-Wert der erfindungsgemäßen Formulierungen im sauren bis sehr schwach alkalischen Bereich kleiner als 8 eingestellt, bevorzugt von 4,0 - 7,5 , besonders bevorzugt von 5,0 - 6,5.

Die jeweils einzusetzenden Mengen an Hilfs- Zusatz- und Trägerstoffen und gegebenenfalls Parfüm können in Abhängigkeit von der Art des jeweiligen Produktes vom Fachmann durch einfaches Ausprobieren leicht ermittelt werden.

Die Herstellung der erfindungsgemäßen dermatologischen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Sollen die erfindungsgemäßen Kombinationen in Puder eingearbeitet werden, so können die Suspensionsgrundlagen dafür vorteilhaft gewählt werden aus der Gruppe

Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die Mengenangaben beziehen sich stets auf Gewichts-%, wenn nichts Anderes angegeben wird. In den Beispielen bedeutet der Begriff "WWS" eine Wollwachssäurefraktion, welche gewonnen wurde aus Rohwollwachssäure durch Kurzwegdestillation im bei 10⁻¹ bar aus dem Destillationstemperaturintervall von 150 - 200° C. Der Anteil an α-Hydroxycarbonsäuren beträgt dabei ca. 22 - 27 %

### Beispiel 1

| Gel Roll-On I - III | | | |
|---|---|---|---|
| | I | II | III |
| PEG-40 Hydriertes Rizinusöl | 1,75 | 1,50 | 1,75 |
| WWS | 0,40 | 0,40 | 0,40 |
| GMC | 0,75 | 0,60 | - |
| GMCy | - | - | 0,90 |
| Ethanol | 62,00 | 62,00 | 60,00 |
| Parfum | q.s. | q.s. | q.s. |
| Wasser, VES | - jeweils ad 100,00 - | | |

### Beispiel 2

| Emulsions Roll-On I - III | | | |
|---|---|---|---|
| | I | II | III |
| PEG-21 Stearylether (Brij 721) | 1,50 | 1,60 | 1,50 |
| PEG-2 Stearylether (Brij 72) | 2,50 | 2,80 | 2,50 |
| Mineralöl DAB 9 | 4,00 | 4,50 | 4,00 |
| Isopropylpalmitat | 3,50 | 3,50 | 4,00 |
| Methyl-Propyl Paraben | 0,15 | 0,15 | 0,15 |
| WWS | 0,70 | 0,70 | 0,70 |
| GMC | 0,70 | - | 0,50 |
| GMCy | - | 0,90 | - |
| Parfum | q.s. | q.s. | q.s. |
| Wasser, VES | - jeweils ad 100,00 - | | |

### Beispiel 3

| Pumpzerstäuber I - II | | |
|---|---|---|
| | I | II |
| Ethanol | 68,00 | 60,00 |
| Propylenglycol-1,2 | 1,80 | 1,80 |
| WWS | 0,30 | 0,30 |
| GMC | 0,70 | - |
| GMCy | - | 1,1 |
| Parfum | q.s. | q.s. |
| Wasser, VES | jeweils ad 100,00 | |

### Beispiel 4

| Gel-Stift I - II | | |
|---|---|---|
| | I | III |
| Stearinsäure | 6,00 | 6,00 |
| Ceteareth-15 | 2,75 | 2,75 |
| WWS | 2,00 | 2,00 |
| GMC | 1,50 | - |
| GMCy | - | 1,60 |
| Ethanol | 16,00 | 16,00 |
| NaOH | 1,10 | 1,05 |
| Parfum | q.s. | q.s. |
| Wasser, VES | - jeweils ad 100,00 - | |

### Beispiel 5

| Deo-Crème I - II | | |
|---|---|---|
| | I | II |
| Mineralöl DAB 9 | 3,50 | 3,50 |
| PEG-40 Stearat | 4,00 | 4,00 |
| Cetylalkohol | 3,50 | 3,50 |
| Ethylhexylstearat | 0,90 | 0,90 |
| Propylenglycol | 1,00 | 1,00 |
| Methyl-propyl-paraben | 0,15 | 0,15 |
| WWS | 0,30 | 0,30 |
| GMC | 0,60 | - |
| GMCy | - | 1,1 |
| Parfum | q.s. | q.s. |
| Wasser, VES | jeweils ad 100,00 | |

### Beispiel 6

| Gel Roll-On IV - VI | | | |
|---|---|---|---|
| | IV | V | VI |
| PEG-40 Hydriertes Rizinusöl | 1,75 | 1,50 | 1,75 |
| WWS | 0,40 | 0,40 | 0,40 |
| DMC | 0,75 | 0,60 | - |
| TML | - | - | 0,90 |
| Ethanol | 62,00 | 62,00 | 60,00 |
| Parfum | q.s. | q.s. | q.s. |
| Wasser, VES | - jeweils ad 100,00 - | | |

### Beispiel 7

| Emulsions Roll-On IV - VI | | | |
|---|---|---|---|
| | IV | V | VI |
| PEG-21 Stearylether (Brij 721) | 1,50 | 1,60 | 1,50 |
| PEG-2 Stearylether (Brij 72) | 2,50 | 2,80 | 2,50 |
| Mineralöl DAB 9 | 4,00 | 4,50 | 4,00 |
| Isopropylpalmitat | 3,50 | 3,50 | 4,00 |
| Methyl-Propyl Paraben | 0,15 | 0,15 | 0,15 |
| WWS | 0,70 | 0,70 | 0,70 |
| DMC | 0,70 | - | 0,50 |
| TML | - | 0,90 | - |
| Parfum | q.s. | q.s. | q.s. |
| Wasser, VES | - jeweils ad 100,00 - | | |

### Beispiel 8

| Pumpzerstäuber III - IV | | |
|---|---|---|
| | III | IV |
| Ethanol | 68,00 | 60,00 |
| Propylenglycol-1,2 | 1,80 | 1,80 |
| WWS | 0,30 | 0,30 |
| DMC | 0,70 | - |
| TML | - | 1,1 |
| Parfum | q.s. | q.s. |
| Wasser, VES | jeweils ad 100,00 | |

### Beispiel 9

| Gel-Stift III - IV | | |
|---|---|---|
| | III | IV |
| Stearinsäure | 6,00 | 6,00 |
| Ceteareth-15 | 2,75 | 2,75 |
| WWS | 2,00 | 2,00 |
| DMC | 1,50 | - |
| TML | - | 1,60 |
| Ethanol | 16,00 | 16,00 |
| NaOH | 1,10 | 1,05 |
| Parfum | q.s. | q.s. |
| Wasser, VES | - jeweils ad 100,00 - | |

### Beispiel 10

| Deo-Crème IV - VI | | |
|---|---|---|
| | IV | VI |
| Mineralöl DAB 9 | 3,50 | 3,50 |
| PEG-40 Stearat | 4,00 | 4,00 |
| Cetylalkohol | 3,50 | 3,50 |
| Ethylhexylstearat | 0,90 | 0,90 |
| Propylenglycol | 1,00 | 1,00 |
| Methyl-propyl-paraben | 0,15 | 0,15 |
| WWS | 0,30 | 0,30 |
| DMC | 0,60 | - |
| TML | - | 1,1 |
| Parfum | q.s. | q.s. |
| Wasser, VES | jeweils ad 100,00 | |

## Patentansprüche

1. Verwendung von Wirkstoffkombinationen aus
I) einem natürlichen Wollwachssäuregemisch oder einem durch Destillation eines natürlichen Wollwachssäuregemisches erhältlichen Wollwachssäuregemisch und
II) einer oder mehrerer Substanzen, gewählt aus der Gruppe der Monoglycerin-monocarbonsäure-monoester, der Diglycerin-monocarbonsäure-monoester und der Triglycerin-monocarbonsäure-monoester
als Wirkprinzip gegen Superinfektionen.

2. Verwendung nach Anspruch 1, dadurch gekennzeichnet, daß das Wollwachssäuregemisch erhältlich ist aus Rohwollwachssäure durch Kurzwegdestillation im Vakuum bei 10⁻¹ bar aus dem Destillationstemperaturintervall von 150 - 200° C.

3. Verwendung nach Anspruch 1, dadurch gekennzeichnet, daß das durch Destillation erhältliche Wollwachssäuregemisch sich durch folgende Parameter auszeichnet:
| | |
|---|---|
| Tropfpunkt | 50 - 54° C |
| Säurezahl | 166 - 170 |
| Verseifungszahl | 175 - 190 |
| OH - Zahl | 60 - 80 |
| Jodzahl | 7 - 12 |

4. Verwendung nach Anspruch 1, dadurch gekennzeichnet, daß der Gehalt an einer oder mehrerer Substanzen, gewählt aus der Gruppe der Monoglycerin-monocarbonsäure-monoester, der Diglycerin-monocarbonsäure-monoester und der Triglycerin-monocarbonsäure-monoester 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung, beträgt.

5. Verwendung nach Anspruch 1, dadurch gekennzeichnet, daß die Substanz oder die Substanzen der Gruppe der Monoglycerin-monocarbonsäure-monoester, der Diglycerin-monocarbonsäure-monoester und der Triglycerin-monocarbonsäure-monoester gewählt wird aus der Gruppe Glycerinmonocaprylat, Glycerinmonocaprinat, Diglycerinmonocaprinat, Triglycerinmonolaurat, Diglycerinmonolaurat Triglycerinmonomyristat .

6. Verwendung nach Anspruch 1, dadurch gekennzeichnet, daß das Verhältnis von der Substanz oder den Substanzen der Gruppe der Monoglycerin-monocarbonsäure-monoester, der Diglycerin-monocarbonsäure-monoester und der Triglycerin-monocarbonsäure-monoester einerseits und dem Wollwachssäuregemisch andererseits zueinander aus dem Bereich von 20 : 1 bis 1 : 20, bevorzugt aus dem Bereich von 5 : 1 bis 1 : 5, gewählt wird.

7. Verwendung von Wirkstoffkombinationen aus
I) einem natürlichen Wollwachssäuregemisch oder einem durch Destillation eines natürlichen Wollwachssäuregemisches erhältlichen Wollwachssäuregemisch und
II) einer oder mehrerer Substanzen, gewählt aus der Gruppe der Monoglycerin-monocarbonsäure-monoester, der Diglycerin-monocarbonsäure-monoester und der Triglycerin-monocarbonsäure-monoester
zur Herstellung einer kosmetischen oder dermatologischen Zubereitung gegen Superinfektionen.
